# EUROPEAN PATENT SPECIFICATION

(11) **EP 0 220 151 B1**
(45) Date of publication and mention of the grant of the patent: **28.04.1993**
(21) Application number: 86850360.8
(22) Date of filing: 21.10.1986
(51) Int. Cl.: A61K 9/00, A61K 9/24

(54) **A particle comprising a coated core**
Fester Körper mit überzogenem Kern
Corps avec un noyau enrobé

(30) Priority: 25.10.1985 SE 8505034
(43) Date of publication of application: 29.04.1987
(73) Proprietor: Aktiebolaget Hässle, S-431 83 Mölndal (SE)
(72) Inventor: Berglund, Bengt Göran, S-413 18 Göteborg (SE)
(74) Representative: Linderoth, Margareta

(56) References cited:
- DE-C- 657 259
- GB-C- 800 973

## Description

The present invention is related to a particle such as a granule, a pellet or a tablet, containing a liquid-soluble active substance for release outside a human or animal body said particle comprising a core with a coating thereon said coating comprising such liquid-soluble active substance. The object of the invention is to provide a visual signal which indicates that the liquid-soluble substance, completely or at a pre-determined part, has been consumed.

The invention has its primary application on granules, tablets and similar drug releasing units which are comprised in a parenteral liquid administration device of the kind shown in Swedish patent application No. 8101247-8. In this case granules or a tablet comprising a drug substance, or another substance suitable for parenteral administration, is placed in a cell through which the liquid flow of a parenteral liquid administration system is flowing, whereby said substance is dissolved by the liquid administred to the patient. In this connection it may be of great value to be able to observe the consumption of the drug to be able to take adequate measures, such as activation of a further drug dosage or disconnection of the liquid supply when a pre-determined amount of the drug has been consumed.

### Description of the invention

According to the invention the core in the particle mentioned initially has an appearance differing from the appearance of the coating, and the appearance of the core is initially invisible by being concealed by the coating and appears only after the dissolution of the liquid-soluble active substance, thus indicating that the active substance in said coating has been dissolved.

Thus, the coating and the core preferably has colours deviating from each other. When the coating contains a drug substance the coating is often white, while the core can be for example blue. The visual difference between the core and the coating can alternatively be achieved for example by different surface structure or different fluoresence properties. It it thus whithin the scope of the invention that the difference in appearance is detectable only in special light or by means of special apparatus, but a difference in appearance that is detectable with the eye in normal light is preferred.

The core is preferably a core insoluble in the liquid, such as a plastic bead or a glas bead which may be dyed with a desired colour or coated with a dye and a protective layer which prevents dissolution in the case that the dye is soluble in the liquid. Said protective layer may consist of a water-insoluble impermeable polymer. In use of such protective layer a core soluble in the liquid can also be used.

The coating can be a coating which contains the liquid-soluble active substance and has an ability to conceal the core, but it is also possible to achieve the concealing property of a coating by means of a layer which is placed inside the layer comprising the liquid soluble substance. Said inner layer can thereby either be soluble in the liquid or have an ability to change from being opaque to being transparent through influence of the liquid.

The layer which comprises the liquid soluble substance can in a manner known per se be provided with release controlling agents, such as a matrix in which the substance is intermixed, for example a polymer matrix where the polymer is insoluble or soluble in the liquid, or an outer membrane comprising a water-insoluble polymer. If said outer membrane is not dissolved by the liquid it will be made of a transparent material or a material which by the influence of the liquid becomes transparent.

The liquid-soluble active substance is preferably a substance suitable for parenteral administration which can be dissolved in a liquid suitable for parenteral administration. Thus, the liquid-soluble active substance may be a drug with healing or alleviating effect, a diagnostic, or a nutrient. Substances useful for continous or intermittent infusion may be comprised. More than one liquid soluble substance may be used in a particle according to the invention. Examples of drugs are antibiotics, cardiovascular agents such as lidocain and β-blockers, cytostatics or gastric acid secretion inhibiting agents. The liquid suitable for parenteral administration is usually an aqueous solution such as an isotonic sodium chloride solution or a dextrose solution. Substances which facilitate the dissolution of liquid soluble substance such as polyethyleneglycol as well as other additives may be comprised.

Particles according to the invention intended to be used in a plurality suitably have the size of 0.5-4 mm.

Preparation of particles according to the invention is suitably done by coating in a fluidized bed, but coating in a coating pan and other techniques for example coacervation may be used. A process for preparation of one or more particles according to the invention is a further aspect of the invention.

Use of one or a plurality of particles according to the invention in a device for parenteral liquid administration, the device mentioned initially, is a further aspect of the invention. Thereby naturally the cell in which the body/bodies is placed will be transparent or designed in another way, thus that the change of appearance may be observed.

In addition to use in parenteral liquid administration the invention can be applied in other technical fields, for example in laboratories, whereby the liquid soluble active substance may be a chemical reagent.

The invention is further described by the following example:
As cores were used 500 g non pareil (sugar-starch beads) fraction 1.18-1.4 mm. These are white, and due to this the beads were coloured blue with 0.13 g methyleneblue in 408 g 95 % ethanol together with 25 g ethylcellulose as an adhesive. An insoluble polymer (25 g ethylcellulose)is then laid on to provent leakage. The active substance consisting of 500 g lidocaine hydrochloride dissolved in 3600 g methylenechloride/isopropanol (50:50 % by weight) were then spraycrystallized on the blue coloured cores which successively turned white. A release controlling film (50 g ethylcellulose disssolved in 2100 g methylenechlorid/isopropanol (75:25 % by weight) was then laid on the granules. The entire process was carried out in a fluidized bed in a 0.5 kg scale. Release tests carried out showed that the granules changed colour from white to blue at the end of the release.

## Claims

1. A particle designed for release of a liquid-soluble active substance outside a human or animal body, said particle comprising a core with a coating thereon, said coating comprising such liquid-solube active substance, **characterized** in that the core has a visual appearance differing from the visual appearance of the coating, and that the core is initially invisible by being concealed by the coating, and becomes visible only after dissolution of the liquid-soluble active substance, thus indicating that the active substance in said coating has been dissolved.

2. A particle according to claim 1, **characterized** in that it has an amount of liquid-soluble active substance adapted for use of the substance in a plurality as granules, pellets or tablets.

3. A particle according to claim 1 and 2, **characterized** in that the liquid-soluble active substance is a substance suitable for parenteral administration which may be dissolved in a liquid suitable for parenteral administration.

4. A particle according to claim 3, **characterized** in that it is a particle designed for release of the active substance in a system for parenteral administration.

5. A particle according to one or more of the of the preceding claims, **characterized** in that the coating comprising a liquid-soluble substance is provided with an agent which controls the release of substance to the liquid.

6. Use of a plurality of particles according to one or more of the preceding claims in a device for parenteral administration.

7. A process for preparation of a particle according to one or more of the preceding claims, **characterized** in providing a core with a coating of liquid-soluble active substance, which coating has an appearance differing from the appearance of the core thus that the core is initially concealed and may be brought to appear after release of the substance, thus indicating that the active substance has been released.

## Patentansprüche

1. Teilchen zur Freisetzung einer flüssigkeitslöslichen aktiven Substanz außerhalb eines menschlichen oder tierischen Körpers, welches Teilchen einen Kern mit einem Überzug darauf aufweist, wobei der Überzug diese flüssigkeitslösliche aktive Substanz enthält, dadurch gekennzeichnet, daß der Kern ein vom optischen Erscheinungsbild des Überzugs abweichendes optisches Erscheinungsbild hat und daß der Kern anfangs unsichtbar ist, indem er durch den Überzug verborgen ist, und erst nach Auflösung der flüssigkeitslöslichen aktiven Substanz sichtbar wird, wodurch angezeigt wird, daß die im Überzug befindliche aktive Substanz aufgelöst worden ist.

2. Teilchen nach Anspruch 1, dadurch gekennzeichnet, daß es eine Menge einer flüssigkeitslöslichen aktiven Substanz aufweist, die zur Verwendung der Substanz in einer Mehrzahl als Granula, Pellets oder Tabletten ausgelegt ist.

3. Teilchen nach Anspruch 1 oder 2, dadurch gekennzeichnet, daß die flüssigkeitslösliche aktive Substanz eine Substanz ist, die zur parenteralen Verabreichung geeignet ist und die in einer zur parenteralen Verabreichung geeigneten Flüssigkeit aufgelöst werden kann.

4. Teilchen nach Anspruch 3, dadurch gekennzeichnet, daß es ein zur Freisetzung der aktiven Substanz in einem System zur parenteralen Verabreichung ausgelegtes Teilchen ist.

5. Teilchen nach einem oder mehreren der vorhergehenden Ansprüche, dadurch gekennzeichnet, daß der eine flüssigkeitslösliche Substanz enthaltende Überzug mit einem Mittel versehen ist, das die Freisetzung der Substanz an die Flüssigkeit steuert.

6. Verwendung einer Mehrzahl von Teilchen nach einem koder mehreren der vorhergehenden Ansprüche in einer Vorrichtung zur parenteralen Verabreichung.

7. Verfahren zur Herstellung eines Teilchens nach einem oder mehreren der vorhergehenden Ansprüche, dadurch gekennzeichnet, daß ein Kern mit einem Überzug einer flüssigkeitslöslichen aktiven Substanz versehen wird, welcher Überzug ein Erscheinungsbild hat, das vom Erscheinungsbild des Kerns abweicht, so daß der Kern anfangs verborgen ist und nach der Freisetzung der Substanz zum Erscheinen gebracht werden kann, wodurch angezeigt wird, daß die aktive Substanz freigesetzt worden ist.

## Revendications

1. Une particule conçue pour la libération d'une substance active soluble dans un liquide à l'extérieur d'un corps humain ou animal, ladite particule comprenant un noyau recouvert d'un enrobage, ledit enrobage contenant cette substance active soluble dans un liquide, caractérisée en ce que le noyau possède un aspect visuel différent de l'aspect visuel de l'enrobage et que le noyau est au départ invisible, masqué par l'enrobage et ne devient visible qu'après dissolution de la substance active soluble dans un liquide, indiquant ainsi que la substance active dans l'enrobage a été dissoute.

2. Une particule selon la revendication 1, caractérisée en ce qu'elle contient une quantité de substance active soluble dans un liquide adaptée à l'emploi de la substance dans une pluralité de particules telles que granulés, pastilles ou comprimés.

3. Une particule selon les revendications 1 et 2, caractérisée en ce que la substance active soluble dans un liquide est une substance convenant à l'administration parentérale qui peut être dissoute dans un liquide convenant à l'administration parentérale.

4. Une particule selon la revendication 3, caractérisée en ce qu'elle est une particule conçue pour la libération de la substance active dans un système destiné à l'administration parentérale.

5. Une particule selon une ou plusieurs des revendications précédentes, caractérisée en ce que l'enrobage qui contient une substance soluble dans un liquide est doté d'un agent qui règle la libération de substance dans le liquide.

6. Utilisation d'une pluralité de particules selon une ou plusieurs des revendications précédentes dans un dispositif destiné à l'administration parentérale.

7. Un procédé pour la préparation d'une particule selon une ou plusieurs des revendications précédentes, caractérisé en ce qu'il fournit un noyau avec un enrobage de substance active soluble dans un liquide, lequel enrobage possède un aspect différent de l'aspect du noyau de manière que le noyau soit masqué au départ et puisse être rendu visible après libération de la substance, indiquant ainsi que la substance active a été libérée.
